# EUROPEAN PATENT SPECIFICATION

(11) **EP 3 326 664 B1**
(45) Date of publication and mention of the grant of the patent: **05.05.2021**
(21) Application number: 17168093.7
(22) Date of filing: 14.05.2014
(51) Int. Cl.: A61M 1/00

(54) **USER INTERLOCK FOR A MEDICAL WASTE FLUID COLLECTION AND DISPOSAL SYSTEM**
BENUTZERVERRIEGELUNG ZUM SAMMELN UND ENTSORGEN VON MEDIZINISCHEN ABFALLFLÜSSIGKEITEN
VERROUILLAGE D'UTILISATEUR POUR LA COLLECTE DE FLUIDES DE DÉCHETS MÉDICAUX ET SYSTÈME D'ÉVACUATION

(30) Priority: 17.05.2013 US 201313896530
(43) Date of publication of application: 30.05.2018
(62) Divisional of application: 14168309.4
(73) Proprietor: Dornoch Medical Systems, Inc., Riverside, MO 64150 (US)
(72) Inventor: SYLVESTER, Jordan B., Riverside, MO 64150 (US); SMITH, Larry C., Riverside, MO 64150 (US); SCHMIDT, Craig B., Riverside, MO 64150 (US)
(74) Representative: Mays, Julie

(56) References cited:
- US-A1- 2006 122 558
- US-A1- 2007 135 779

## Description

### TECHNICAL FIELD

The present disclosure relates generally to systems and methods for collecting biological fluids during medical procedures and, in particular, to a user interlock screen for verifying a selected vacuum setting for use when collecting biological fluids.

### BACKGROUND

Biological fluids and other types of medical waste often must be collected during surgery or other medical procedures (e.g. US 2006/122558 A1, US 2007/135779 A1). This is typically accomplished using a medical waste fluid collection cart, which may be part of a medical waste fluid collection and disposal system. Such carts may include at least one suction canister where a suction port on the canister lid is connected to a source of vacuum or suction via a hose or line. As a result, a vacuum is drawn on the interior of the canister. A second hose or line is connected to a "patient" port on the canister lid and is used to collect medical waste in the form of fluids and solids from the patient, which is stored in the canister. After collection, the medical waste and contaminated collection components, such as canister lids, and the like, must be disposed of in accordance with rules and regulations imposed by various government and regulatory organizations.

In some cases, medical waste fluid collection carts may be designed for use in a variety of different of medical procedures. For example, a medical waste fluid collection cart may be used in a surgical setting involving large amounts of fluid, such as arthroscopic knee surgery. In such cases, the user may desire to operate the medical waste fluid collection cart at a high flow rate that may be about 300 standard cubic feet per hour (SCFH) or more, and may involve a high vacuum level (e.g., about 600 mm Hg). In other cases, such as in low flow and/or passive collection applications, a high flow rate and/or high vacuum level may have adverse consequences. For example, during a surgical procedure associated with delicate tissues (e.g., brain surgery) or during a medical procedure involving a closed body cavity (e.g., a chest tube) where the closed body cavity is receiving the vacuum, only very low vacuum settings (e.g. less than about 50 mm Hg) and extremely low flow may be desired. Because of the broad range of vacuum settings and/or flow level requirements over the range of possible medical procedures in which the medical waste fluid cart may be used, it may be advantageous to provide a user interlock to enable a user to verify at least one of a flow rate setting and/or a vacuum level setting prior to activating the medical waste fluid collection cart.

### SUMMARY

The invention is defined in the appended claims.

The present disclosure relates generally to systems and methods for collecting biological fluids during medical procedures and, in particular, to a user interlock screen to verify a selected vacuum setting for use in collecting biological fluids using a medical fluid collection system. In some cases, a medical waste fluid collection cart for use in a medical waste fluid collection and disposal system may include a graphical user interface, wherein the medical waste fluid collection cart may include a controller for controlling one or more functions of the medical waste fluid collection cart and a memory coupled to the controller, the memory configured for storing one or more vacuum levels. The graphical user interface may be communicatively coupled to the controller and/or the memory and may be configured to allow a user to monitor and/or control one or more functions of the medical waste fluid collection cart, wherein the graphical user interface may include at least one user confirmation screen to allow a user to confirm a vacuum setting. Using the confirmation screen, the user may confirm the vacuum setting using a first selection corresponding to a predetermined low vacuum level or a second selection corresponding to a previously used vacuum level.

According to one example there is provided a medical waste fluid collection cart including a graphical user interface for allowing a user to operate or monitor one or more functions of the medical waste fluid collection cart, the medical waste fluid collection cart comprising:
at least one fluid collection cylinder;
a manifold configured to allow for fluid transfer into and/or out of the at least one fluid collection cylinder;
a suction tube in fluid communication with the at least one fluid collection cylinder, the suction tube for providing a fluid path to the at least one fluid collection cylinder for medical waste fluid;
a vacuum port in fluid communication with the at least one fluid collection cylinder, the vacuum port configured to connect the at least one fluid collection cylinder to a vacuum source to provide a vacuum level in the fluid collection cylinder for use in collecting a medical waste fluid, wherein the vacuum level in the at least one fluid collection cylinder is adjustable;
a controller for controlling one or more functions of the medical waste fluid collection cart including providing a command to adjust the vacuum level provided at the vacuum port, wherein the controller may enable a particular vacuum level upon power up of the medical waste fluid collection cart;
a memory coupled to the controller, the memory configured for storing one or more vacuum levels;
a graphical user interface communicatively coupled to the controller and/or the memory, the graphical user interface configured to allow a user to monitor and/or control one or more functions of the medical waste fluid collection cart, wherein the graphical user interface includes at least one user confirmation screen to allow a user to confirm a vacuum setting, the screen including:
   a first selection corresponding to a predetermined low vacuum level; and
   a second selection corresponding to a previously used vacuum level.

In some cases, a method of providing a user interlock for a medical waste fluid collection and disposal system may include initiating a startup procedure of the medical waste fluid collection and disposal system. Power may be applied to a portion of the medical waste fluid collection and disposal system, such as a medical waste fluid collection cart, or may cycle power to a portion of the medical waste fluid collection and disposal system. The medical waste fluid collection cart may also be configured for determining whether an improper shutdown of the medical waste fluid collection and disposal system preceded the initiated startup procedure. In some cases, electrical power may be restored to the medical waste fluid collection cart after electrical power was interrupted. Occasionally, power may be interrupted (e.g., a disconnected power cord, an electrical power outage, etc.) during a medical waste fluid collection process and/or without resetting one or more configuration settings following completion of a medical waste fluid collection process. If an improper shutdown of the medical waste fluid collection and disposal system is identified, the medical waste fluid collection cart may be configured for displaying a notification to a user during the startup procedure, the notification prompting the user to select a vacuum level for use by the medical fluid waste collection and disposal system by selecting either a predetermined vacuum level or a previously programmed vacuum level associated with a previous fluid collection process. For example, the medical waste fluid collection cart may be configured to include a preprogrammed vacuum setting and/or flow rate for use with a number of medical procedures or may be configured to store a last known vacuum level and/or flow rate or other previously programmed vacuum level and/or flow rate.

The controller may be included within the user interface. In other cases, one or more components of the controller may be provided and/or mounted on the medical waste fluid collection cart separate from the user interface. The controller may include a processor (e.g. microprocessor, microcontroller, etc.) that may be communicatively coupled via a data bus to one or more components of the controller including one or more units of memory, an input/output block , a data port, a user interface and/or a communication interface. In some cases, the user interface may be configured to display one or more screens to a user.

The processor may operate using a control algorithm that controls or at least partially controls the collection of medical waste fluid and/or disposal of the collected medical waste fluid. The processor may, for example, operate in accordance with an algorithm for collecting medical waste fluid during a medical procedure using one or more vacuum levels and/or fluid flow rates that may be specified by a user and/or may be preset in the memory. In some cases, the instructions may allow the processor to implement a method of providing a user interlock for the medical waste fluid collection cart of the medical waste fluid collection and disposal system. For example, the processor may be configured to initiate a startup procedure of the medical waste fluid collection and disposal system upon powering on the controller . For example, upon receiving power the processor may process instructions for initializing and/or otherwise preparing the medical waste fluid collection cart for use during a particular medical procedure. The processor may be configured to determine whether an improper shutdown of one or more components (e.g., the medical waste fluid collection cart, the evacuation station, etc.) of the medical waste fluid collection and disposal system immediately preceded the initiated startup procedure. An example of a startup procedure may include a first startup procedure for use during normal startup conditions and a second startup procedure for use after an improper shutdown procedure (e.g., loss of electrical power, a failure to reset a parameter setting, etc.). In some instances, the processor may be configured to initiate vacuum at a predetermined and/or otherwise preset vacuum level (or flow rate), such as at a low vacuum level, upon start up of the medical waste fluid collection cart, during normal startup conditions, after an improper shutdown procedure, and/or regardless of the previous usage.

For example, an improper shutdown of the medical waste fluid collection cart and/or the evacuation station of the medical waste fluid collection and disposal system may include a loss of power (e.g., electrical power) to the medical waste fluid collection cart and/or the evacuation station. In some cases, an improper shutdown may be indicated after a failure to reset a parameter setting (e.g., a fluid volume setting) before removing electrical power from the components of the medical waste fluid collection and disposal system. In other cases, an improper shutdown may include a failure to complete a previously started medical waste fluid collection process. In some cases, the processor may be configured to process an algorithm for monitoring the incoming power to predict or otherwise detect an imminent or active power outage.

For such cases, the processor may be configured to store a vacuum level into a location in the memory where the stored vacuum level corresponds to the vacuum level that was in use immediately before power was disconnected from the medical waste fluid collection and disposal system. If an improper shutdown of a component of the medical waste fluid collection and disposal system was identified, the processor may provide information to be displayed in a notification on the user interface to a user during the subsequent startup procedure. The notification may prompt the user to verify a vacuum level for use by the medical fluid waste collection and disposal system by selecting either, for example, a predetermined/preset vacuum level or a previously programmed vacuum level associated with a previous fluid collection process, where one or more of the predetermined/preset vacuum level or the previously programmed vacuum level are provided by the processor and may be read from the memory. Of course, the notification screen may also be displayed to a user during the normal startup procedure where the indication of an improper shutdown is not detected, if desired.

In some cases, the notification screen may be used to inform a user that a predetermined and/or otherwise preset vacuum level (or flow rate) will be used upon start up of the medical waste fluid collection cart. Sometimes, a notification may not be displayed to a user after every startup. In such cases, the medical waste fluid collection cart may be configured to use a predetermined default vacuum level (e.g., at or near zero mm Hg), after a successful startup procedure until the user enters or selects a different vacuum level or flow rate using an input device (e.g., the touch screen, a keyboard, a rotary dial, etc.). In such cases, the input device may be used to provide the user interlock mechanism in preventing the wrong vacuum level or flow rate from being used with a particular medical procedure.

In some cases, the processor may be configured to store a vacuum level associated with an active medical waste fluid collection process in the memory. The vacuum level may be stored before the medical waste fluid collection process begins, while the medical waste fluid collection process is active, or after the medical waste fluid collection cart loses power while the medical waste fluid collection process is active. The processor may also store into the memory an indication (e.g., a flag, a binary value, an integer value, etc.) whether the active medical waste fluid collection process completes successfully. The processor may use the stored indication of successful completion of the active waste fluid collection process to display, or not to display (e.g., bypass), the user notification screen before beginning a subsequent medical waste fluid collection process. In some cases, the stored indication may be used to determine which information (e.g., stored vacuum levels, stored flow rate values, etc.) is to be displayed on the user notification screen and/or which information is to be prompted to be received from the user.

The processor may be configured to process instructions to allow for user confirmation of a selected vacuum level for a medical waste fluid collection and disposal system prior to turning on the vacuum pump or otherwise creating suction in the waste canister or cylinder and/or suction tubing to the patient. For example, the processor may be configured for obtaining a vacuum level for use during a medical waste fluid collection process using a user interface of the medical waste fluid collection cart. The processor may obtain the vacuum level via a user input device and/or may obtain the vacuum level from a memory. The processor may then provide an indication of the obtained vacuum level to a user via a user interface, such as via the user interface screens. The user interface screens may be configured to prompt the user for confirmation of the obtained vacuum level before initiating the medical waste fluid collection process (e.g., before turning on the vacuum pump or otherwise creating suction in the waste canister or cylinder and/or suction tubing to the patient) and/or for prompting the user to enter a different vacuum level and/or flow rate to be used during the medical waste fluid collection process.

For example, the processor may be configured for reading two or more vacuum levels and/or flow rates from a memory or for obtaining the two or more vacuum levels and/or flow rates from a different source, such as a user interface screen and/or a manual user interface device (e.g., a switch, a potentiometer, dial, etc.). A first vacuum level may be read from the memory wherein the first vacuum level is a vacuum level which may correspond to a passive fluid collection process. A second vacuum level may be read from the memory wherein the second vacuum level may have been stored by the processor during a previously initiated medical waste fluid collection process (which, in some instances, may have been interrupted by a loss of electrical power). A third vacuum level and/or flow rate may be manually entered by a user via a graphical user interface (e.g., a touchscreen, an LED display, an LCD display, a CRT display, etc.) and/or an electromechanical user interface device (e.g., a switch, a potentiometer, dial, etc.). Once entered, the processor may receive the confirmed and/or selected vacuum level from the user interface for use in a subsequent medical waste fluid collection process.

In some cases, the memory may be used to store predetermined/preset vacuum levels and/or flow rates. In some cases, the stored predetermined/preset vacuum levels and/or flow rates may be associated with one or more medical waste fluid collection processes, such as one or more passive fluid collection processes (e.g., near zero mm Hg, about 90 mm Hg or less, about 80 mm Hg or less, about 75 mm Hg or less, about 60 mm Hg or less, about 50 mm Hg or less, about 30 mm Hg or less, within a range from about 0 mm Hg to about 90 mm Hg, a range of about 0 mm Hg to about 75 mm Hg, a range of about 5 mm Hg to about 90 mm Hg, a range of about 5 mm Hg to about 75 mm Hg, a range of about 5 mm Hg to about 50mm Hg, etc.) or one or more higher rate fluid collection processes (e.g., about 100 mm Hg or more, about 200 mm Hg or more, about 300 mm Hg or more, within a range from about 100 mm Hg to about 350 mm Hg, etc).

Similarly, the medical waste fluid collection and disposal system may be configured to implement another method for user confirmation of a selected vacuum level for a medical waste fluid collection and disposal system including obtaining a vacuum level for use during a medical waste fluid collection process using a user interface of the medical waste fluid collection and disposal system, providing an indication of the obtained vacuum level to a user via a user interface, and prompting the user for confirmation of the obtained vacuum level before initiating the medical waste fluid collection process. In some cases the medical waste fluid collection cart may be configured for reading a first vacuum level from a memory, wherein the first vacuum level is a vacuum level corresponding to a passive fluid collection process and/or reading a second vacuum level from the memory, wherein the second vacuum level was stored by a controller during a previously initiated medical waste fluid collection process. For example, a medical waste fluid collection cart may obtain the vacuum level from a memory location, such as a memory location storing a preprogrammed vacuum level (e.g., 0 mm Hg, 50 mm Hg, etc.), a memory location storing a last known vacuum level, or a user prompt on a user interface screen. When displaying an image to a user, the image may include an indication of the first vacuum level associated with passive fluid collection and/or an indication of the second vacuum level associated with the previously initiated medical waste fluid collection process. In some cases, the user interface may provide one or more input mechanisms for prompting the user for a desired vacuum level associated with a desired fluid collection process.

The user interface screen may be configured to solicit information (e.g., a vacuum level, a flow rate, etc.) from a user as part of a user interlock procedure upon power-up of the medical waste fluid collection cart. In a data solicitation section of the screen, one or more selection fields or buttons may be presented to a user, for example. For example, the user interface screen may be used for providing a user interface to facilitate user entry of one or more vacuum levels for use by the medical waste fluid collection and disposal system. The user interface screen may be configured to receive a user selected vacuum level via the user interface, followed by storing the user entered vacuum level in a memory for use by the medical waste fluid collection and disposal system.

The selection fields or buttons may be associated with possible selections available to the user. For example, the first selection field or button may correspond to a first vacuum level, such as a vacuum level associated with a passive collection operation. The selection field or button may include descriptive text and/or an indication of an associated vacuum level (e.g., a vacuum level within the range of about 0 mm Hg to about 70 mm Hg). The second selection field or button may correspond to a second vacuum level, such as a vacuum level associated an immediately preceding use of the medical fluid collection cart. Similarly, the selection field or button may include descriptive text and/or an indication corresponding to a vacuum level associated with the previously programmed vacuum level (e.g., the last known vacuum level used prior to an error condition). In some cases, only one selection field may be shown, such as when it has been decided to use a predetermined vacuum level after power has been applied to the medical waste fluid collection cart. In such cases, the selection field 340 may display the value of the predetermined vacuum level.

### BRIEF DESCRIPTION OF THE DRAWINGS

The disclosure may be more completely understood in consideration of the following description of various illustrative embodiments in connection with the accompanying drawings, in which:
FIG. 1 is a perspective view of a medical waste fluid collection and disposal system including a medical waste fluid collection cart.
FIG. 2 is a block diagram representation of an illustrative controller for the medical waste fluid collection cart of FIG. 1;
FIG. 3 shows an illustrative user interface screen for providing a user interlock for the medical waste fluid collection cart of FIG. 1;
FIG. 4 shows an illustrative technique of providing a user interlock for configuring a vacuum level setting in the medical waste fluid collection cart of FIG. 1.

While the disclosure is amenable to various modifications and alternative forms, specifics thereof have been shown by way of example in the drawings and will be described in detail. It should be understood, however, that the intention is not to limit aspects of the disclosure to the particular illustrative embodiments described. On the contrary, the intention is to cover all modifications, equivalents, and alternatives falling within the spirit and scope of the disclosure.

### DESCRIPTION

The following description should be read with reference to the drawings wherein like reference numerals indicate like elements throughout the several views. The description and drawings show several illustrative embodiments which are meant to illustrative in nature.

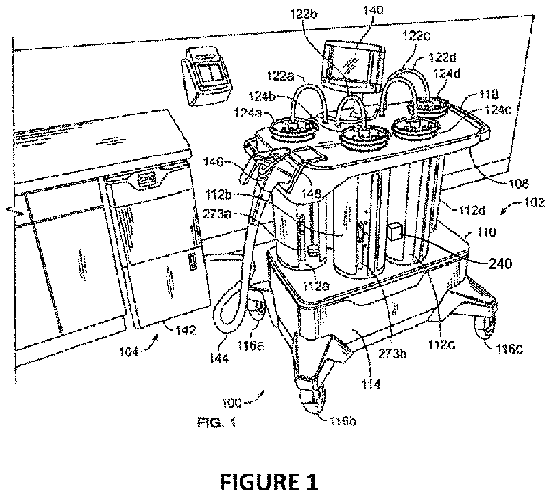

FIG. 1 is a perspective view of a medical waste fluid collection and disposal system 100 including a medical waste fluid collection cart 102 and an evacuation station 104. An illustrative medical waste fluid collection and disposal system, which is not meant to be limiting in any way, is disclosed in, for example: U.S. Patent No: 8,292,857 filed on October 6, 2008, entitled "MEDICAL WASTE FLUID COLLECTION AND DISPOSAL SYSTEM". In some cases the medical waste fluid collection cart 102 may include a top plate 108 and a bottom plate 110. Positioned between the top and bottom plates are fluid collection canisters or cylinders 112a-112d. While four cylinders are shown in FIG. 1, the medical waste fluid collection cart 102 may include an alternative number of cylinders, for example, the medical waste fluid collection cart 102 may be configured to include one cylinder, two cylinders, or another number of cylinders, if desired. The medical waste fluid collection cart 102 also may include a bottom cabinet 114 that may be mounted on casters 116a-116d. The casters 116a-116d may be used to provide steering capability for the cart. The casters 116a-116d may include a brake feature. The top plate 108 features a handle 118. As a result, the medical waste fluid collection cart 102may be easily pushed to and from an operating room.

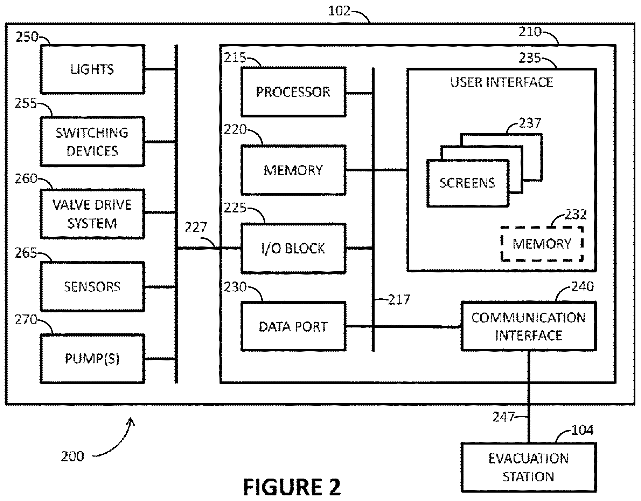

In some cases, each of the cylinders 112a-112d may be connected between a number of corresponding lids 124a-124d via flexible tubing 122a-122d to a vacuum source. The lids may be constructed of molded plastic and may be disposable. The flexible tubing 122a of FIG. 1 may attach at one end to a vacuum port of the lid 124a, with the opposite end attaching to a vacuum pump. The vacuum pump may be provided on the cart 102, or the vacuum tubing may be connected to a vacuum source provided in the medical facility, such as a vacuum pump associated with wall suction provided in an operating room. The vacuum port may be provided with a filter, such as a hydrophobic filter, positioned in compartment in the lid 124a. A patient suction tube may be connected to a suction port on the lid 124a, while one or more additional ports may be covered by caps. The lid 124a also may include a tubing post for occluded vacuum with the patient tubing. The same applies for the remaining cylinder lids 124b-124d of FIG. 1. As a result, vacuum or suction is selectively drawn on each cylinder so that fluids may be collected therein during the medical procedure via the suction tubing extending from the cylinder to the patient. The suction and other functions of the medical waste fluid collection cart 102 may be controlled via a user interface 140 (e.g., a touch screen), which may include and/or may be communicably coupled to the controller of FIG. 2.

After the medical waste fluid collection cart 102 is used in an operating room procedure, and fluids have been collected in one or more of the cylinders 112a-112d, the patient suction tubing is removed from the cylinder lids and all ports for each cylinder lid 124 to which suction was applied are covered with caps. The medical waste fluid collection cart 102 may then be rolled to a position adjacent to the evacuation station 104 for draining, washing, disinfecting, rinsing and return to a suction collection state, as illustrated in FIG. 1.

The evacuation station 104 may include a housing 142 that contains a source of disinfection solution, pump, drainage system and other components, which are described below, for draining washing and disinfecting the cylinders of the medical waste fluid collection cart 102. The evacuation station 104 communicates with the medical waste fluid collection cart 102 by way of the composite hose 144 and the coupler 146. As illustrated in FIG. 1, the coupler 146 is received within a receptacle 148 of the medical waste fluid collection cart 102. Washing fluid from the evacuation station 104 may be dispensed in the cylinders 112 with cleaning nozzles 273 (nozzles 273a, 273b shown) positioned in the cylinders 112 to wash and disinfect the cylinders 112.

In some cases, the medical waste fluid collection cart may include a "wash" valve system to facilitate washing and/or disinfecting the cylinders 112a-d, a "drain" valve system to facilitate draining fluids from the cylinders 112a-d and/or a "vent" valve system to facilitate venting of the cylinders 112a-d. The wash valve system, the drain valve system and/or the vent valve system may include one or more valves, one or more valve drive motors for controlling the operation of the valves, one or more tubes for providing fluid transfer to and/or from the cylinders 112a-d and one or more electrical components for controlling the operation of the valves and/or valve drive motors. A fluid manifold 240 may be positioned between two or more cylinders 112a-112d and may extend into the interior of the cabinet 114. The fluid manifold 240 may include a "wash" tube fitting, a "drain" tube fitting and a "vent" tube fitting. The "wash" tube fitting may communicate with a 3-way "wash" valve and/or the cleaning nozzles 273a, 273b via a fluid passage. The "drain" tube fitting may similarly communicate with a 3-way "drain" valve, via a fluid passage. The "vent" tube fitting may similarly communicate with a 3-way "vent" valve.

When the draining, washing and disinfecting of the cart cylinders 112a-112d is complete, the coupler 146 is removed from the receptacle 148 on the medical waste fluid collection cart 102 so that the medical waste fluid collection cart 102 may again be rolled to an operating room for use. The coupler 146 permits a single connection to be made between the medical waste fluid collection cart 102 and the evacuation station 104. This simplifies and expedites connecting the medical waste fluid collection cart 102 to, and disconnecting the medical waste fluid collection cart 102 from, the evacuation station 104. When the medical waste fluid collection cart 102 and the evacuation station 104 are connected using the coupler 146, one or more communication links may be used to permit a controller of the medical waste fluid collection cart 102 and a controller of the evacuation station 104 to communicate so that the user can control both, including one or more of a draining operation, a washing operation and a disinfecting operation, via the touch screen 140.

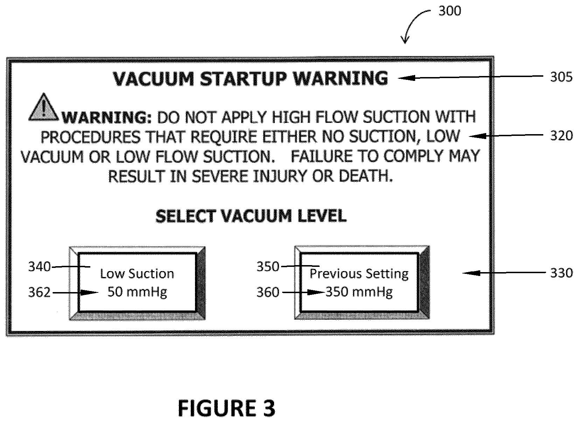

FIG. 2 is a block diagram representation of an illustrative controller 210 for the medical waste fluid collection cart 102 of FIG. 1. In some cases, the medical waste fluid collection cart 102 may include one or more components capable of processing instructions for controlling one or more functions of the medical waste fluid collection cart 102. In some cases, the controller 210 may be included within the user interface 140 of FIG. 1. In other cases, one or more components of the controller 210 may be provided and/or mounted on the medical waste fluid collection cart 102 separate from the user interface 140. The controller 210 may include a processor 215 (e.g. microprocessor, microcontroller, etc.) that may be communicatively coupled via a data bus 217 to one or more components of the controller 210 including one or more units of memory 220, 232, an input/output block 225, a data port 230, a user interface 235 and/or a communication interface 240. In some cases, the user interface 235 may be configured to display one or more screens 237 to a user, such as the example of a user interface screen 300 of FIG. 3.

The processor 215 may operate using a control algorithm that controls or at least partially controls the collection of medical waste fluid and/or disposal of the collected medical waste fluid. The processor 215 may, for example, operate in accordance with an algorithm for collecting medical waste fluid during a medical procedure using one or more vacuum levels and/or fluid flow rates that may be specified by a user and/or may be preset in the memory 220, 232, for example. In some cases, the instructions may allow the processor 215 to implement a method of providing a user interlock for the medical waste fluid collection cart 102 of the medical waste fluid collection and disposal system 100. For example, the processor 215 may be configured to initiate a startup procedure of the medical waste fluid collection and disposal system 100 upon powering on the controller 210. For example, upon receiving power the processor 215 may process instructions for initializing and/or otherwise preparing the medical waste fluid collection cart 102 for use during a particular medical procedure. The processor 215 may be configured to determine whether an improper shutdown of one or more components (e.g., the medical waste fluid collection cart 102, the evacuation station 104, etc.) of the medical waste fluid collection and disposal system 100 immediately preceded the initiated startup procedure. An example of a startup procedure may include a first startup procedure for use during normal startup conditions and a second startup procedure for use after an improper shutdown procedure (e.g., loss of electrical power, a failure to reset a parameter setting, etc.). In some instances, the processor 215 may be configured to initiate vacuum at a predetermined and/or otherwise preset vacuum level (or flow rate), such as at a low vacuum level, upon start up of the medical waste fluid collection cart 102, during normal startup conditions, after an improper shutdown procedure, and/or regardless of the previous usage.

For example, an improper shutdown of the medical waste fluid collection cart 102 and/or the evacuation station 104 of the medical waste fluid collection and disposal system 100 may include a loss of power (e.g., electrical power) to the medical waste fluid collection cart 102 and/or the evacuation station 104. In some cases, an improper shutdown may be indicated after a failure to reset a parameter setting (e.g., a fluid volume setting) before removing electrical power from the components of the medical waste fluid collection and disposal system 100. In other cases, an improper shutdown may include a failure to complete a previously started medical waste fluid collection process. In some cases, the processor 215 may be configured to process an algorithm for monitoring the incoming power to predict or otherwise detect an imminent or active power outage.

For such cases, the processor 215 may be configured to store a vacuum level into a location in the memory 220, 232, where the stored vacuum level corresponds to the vacuum level that was in use immediately before power was disconnected from the medical waste fluid collection and disposal system 100. If an improper shutdown of a component of the medical waste fluid collection and disposal system 100 was identified, the processor 215 may provide information to be displayed in a notification on the user interface 235 to a user during the subsequent startup procedure. The notification may prompt the user to verify a vacuum level for use by the medical fluid waste collection and disposal system 100 by selecting either, for example, a predetermined/preset vacuum level or a previously programmed vacuum level associated with a previous fluid collection process, where one or more of the predetermined/preset vacuum level or the previously programmed vacuum level are provided by the processor 215 and may be read from the memory 220, 232. Of course, the notification screen may also be displayed to a user during the normal startup procedure where the indication of an improper shutdown is not detected, if desired.

In some cases, the notification screen may be used to inform a user that a predetermined and/or otherwise preset vacuum level (or flow rate) will be used upon start up of the medical waste fluid collection cart. Sometimes, a notification may not be displayed to a user after every startup. In such cases, the medical waste fluid collection cart may be configured to use a predetermined default vacuum level (e.g., at or near zero mm Hg), after a successful startup procedure until the user enters or selects a different vacuum level or flow rate using an input device (e.g., the touch screen, a keyboard, a rotary dial, etc.). In such cases, the input device may be used to provide the user interlock mechanism in preventing the wrong vacuum level or flow rate from being used with a particular medical procedure.

In some cases, the processor 215 may be configured to store a vacuum level associated with an active medical waste fluid collection process in the memory 220, 232. The vacuum level may be stored before the medical waste fluid collection process begins, while the medical waste fluid collection process is active, or after the medical waste fluid collection cart 102 loses power while the medical waste fluid collection process is active. The processor 215 may also store into the memory 220, 232 an indication (e.g., a flag, a binary value, an integer value, etc.) whether the active medical waste fluid collection process completes successfully. The processor 215 may use the stored indication of successful completion of the active waste fluid collection process to display, or not to display (e.g., bypass), the user notification screen before beginning a subsequent medical waste fluid collection process. In some cases, the stored indication may be used to determine which information (e.g., stored vacuum levels, stored flow rate values, etc.) is to be displayed on the user notification screen and/or which information is to be prompted to be received from the user.

The processor 215 may be configured to process instructions to allow for user confirmation of a selected vacuum level for a medical waste fluid collection and disposal system 100 prior to turning on the vacuum pump or otherwise creating suction in the waste canister or cylinder 112 and/or suction tubing to the patient. For example, the processor 215 may be configured for obtaining a vacuum level for use during a medical waste fluid collection process using a user interface 140, 235 of the medical waste fluid collection cart 102. The processor 215 may obtain the vacuum level via a user input device and/or may obtain the vacuum level from a memory 220, 232. The processor 215 may then provide an indication of the obtained vacuum level to a user via a user interface 235, such as via the user interface screens 237. The user interface screens 237 may be configured to prompt the user for confirmation of the obtained vacuum level before initiating the medical waste fluid collection process (e.g., before turning on the vacuum pump or otherwise creating suction in the waste canister or cylinder 112 and/or suction tubing to the patient) and/or for prompting the user to enter a different vacuum level and/or flow rate to be used during the medical waste fluid collection process.

For example, the processor 215 may be configured for reading two or more vacuum levels and/or flow rates from a memory 220, 232 or for obtaining the two or more vacuum levels and/or flow rates from a different source, such as a user interface screen and/or a manual user interface device (e.g., a switch, a potentiometer, dial, etc.). A first vacuum level may be read from the memory 220, 232, wherein the first vacuum level is a vacuum level which may correspond to a passive fluid collection process. A second vacuum level may be read from the memory 220, 232, wherein the second vacuum level may have been stored by the processor 215 during a previously initiated medical waste fluid collection process (which, in some instances, may have been interrupted by a loss of electrical power). A third vacuum level and/or flow rate may be manually entered by a user via a graphical user interface (e.g., a touchscreen, an LED display, an LCD display, a CRT display, etc.) and/or an electromechanical user interface device (e.g., a switch, a potentiometer, dial, etc.). Once entered, the processor 215 may receive the confirmed and/or selected vacuum level from the user interface 235 for use in a subsequent medical waste fluid collection process.

In one example, the processor 215 may be configured to operate the algorithm using an operating system (e.g., Windows, OS X, iOS, Android, Linux, Unix, GNU, etc.), or an example embedded operating system (e.g., QNX, NiagaraAX, Windows CE, etc.). In some cases, the controller 210 may include a timer (not shown). The timer may be integral to the processor 215 or may be provided as a separate component.

The memory 220, 232 of the illustrative controller 210 may be communicatively coupled to the processor 215. The memory 220, 232 may be used to store any desired information, such as the aforementioned control algorithm, a power monitoring algorithm, the configuration of the medical waste fluid collection and disposal system 100, set points, vacuum levels, flow levels, flags, indicators, diagnostic limits, and/or the like. The memory 220, 232 may be any suitable type of storage device including, but not limited to, RAM, ROM, EPROM, flash memory, a hard drive, and/or the like. In some cases, the processor 215 may store information within memory 220, 232, and may subsequently retrieve the stored information. In some cases, the memory 220, 232 may be used to store predetermined/preset vacuum levels and/or flow rates. In some cases, the stored predetermined/preset vacuum levels and/or flow rates may be associated with one or more medical waste fluid collection processes, such as one or more passive fluid collection processes (e.g., near zero mm Hg, about 90 mm Hg or less, about 80 mm Hg or less, about 75 mm Hg or less, about 60 mm Hg or less, about 50 mm Hg or less, about 30 mm Hg or less, within a range from about 0 mm Hg to about 90 mm Hg, a range of about 0 mm Hg to about 75 mm Hg, a range of about 5 mm Hg to about 90 mm Hg, a range of about 5 mm Hg to about 75 mm Hg, a range of about 5 mm Hg to about 50mm Hg, etc.) or one or more higher rate fluid collection processes (e.g., about 100 mm Hg or more, about 200 mm Hg or more, about 300 mm Hg or more, within a range from about 100mm Hg to about 350mm Hg, etc.).

In some cases, the controller 210 may include an input/output block (I/O block) 225 for receiving one or more signals from one or more components of the medical waste fluid collection cart 102 and or for providing one or more signals to the one or more components of the medical waste fluid collection cart 102. For example, the I/O block 225 may be configured to provide signals to and/or to receive signals from one or more lights 250 (e.g., cylinder lights associated with the cylinders 112a-d), one or more switching devices 255 (e.g., solenoids, relays, transistors, etc.), one or more components of a valve drive system 260 (e.g., motors, valves, etc.) including one or more components of the "wash" valve, the "drain" valve and/or the "vent" valve, one or more sensors 265 (e.g., pressure sensors, level sensors, flow sensors, etc.), pumps 270 (e.g., a vacuum pump, an evacuation pump, etc.), and/or one or more other components via one or more communication paths 227. The I/O block 225 may be configured for wired communication via one or more terminal screws, for example, and/or wireless communication via a wireless communication interface, for example. In some cases, the I/O block 225 may be used to communicate with other sensors and/or other devices associated with a particular medical process.

In some cases, as illustrated in FIG. 2, the controller 210 may include a data port 230. The data port 230 may be a wireless port such as a Bluetooth™, WiFi, Zigbee or any other wireless protocol. In other cases, data port 230 may be a wired port such as a serial port, an ARCNET port, a parallel port, a CAT5 port, a USB (universal serial bus) port, and/or the like. In some cases, the data port 230 may use one or more communication protocols, such as Ethernet, BACNet, LONtalk, etc., that may be used via a wired network or a wireless network. In some instances, the data port 230 may be a USB port and may be used to download and/or upload information from a USB flash drive or some other data source. Other remote devices may also be employed, as desired.

The data port 230 may be configured to communicate with the processor 215 and may, if desired, be used to upload information to the processor 215 and/or download information from the processor 215. Information that can be uploaded and/or downloaded may include, for example, values of operating parameters (e.g., vacuum levels, flow rates, etc.). In some instances, the data port 230 may be used to upload previously-created configurations and/or software updates into the controller 210, thereby hastening the programming process.

In some cases, the data port 230 may be used to download data stored within the memory 220, 232 for analysis and/or transfer to another device. For example, the data port 230 may be used to download, one or more stored flow rates and/or vacuum levels, a faults and/or alerts log and/or parts thereof to a remote device such as a USB memory stick (also sometimes referred to as a thumb drive or jump drive), personal computer, laptop, iPAD® or other tablet computer, PDA, smart phone, or other device, as desired. In some cases, the data may be convertible to an MS EXCEL®, MS WORD®, text, XML, and/or Adobe PDF® file, if desired.

In the illustrative embodiment of FIG. 2, the user interface 235 may be any suitable user interface that permits the controller 210 to display and/or solicit information, as well as accept one or more user interactions with the controller 210. For example, the user interface 235 may permit a user to enter data such as vacuum levels, flow rates, medical application types, and the like. In some cases, the user interface 235 may include a display and a distinct keypad. A display may be any suitable display. In some instances, the display may include or may be a liquid crystal display (LCD), and in some cases a fixed segment display or a dot matrix LCD display. If desired, the user interface 235 may be a touch screen LCD panel that functions as both display and keypad. In some instances, a touch screen LCD panel may be adapted to solicit values for a number of operating parameters and/or to receive such values, if desired. In some cases, the user interface 235 may optionally include the memory 232. In some cases, the user interface 235 may include one or more electromechanical input devices (e.g., a switch, a potentiometer, a rotary dial, a push button, etc.) for use in selecting a desired flow rate and/or vacuum level.

For example, the user interface 235 may include a graphical user interface communicatively coupled to the processor 215 and/or the memory 220, 232 via the data bus 217. The user interface 235 may be configured to allow a user to monitor and/or control one or more functions of the medical waste fluid collection cart 102 and/or the evacuation station 104. The user interface 235 may include one or more screens 237 that may be used to present information to a user and may include at least one user confirmation screen (e.g., the user interface screen 300 of FIG. 3) to allow a user to select and/or confirm a vacuum setting. For example the user confirmation screen may be used to prompt or otherwise solicit user confirmation of a vacuum level and/or a flow rate to be used on a subsequent medical waste fluid collection process. In some cases, a graphical user interface may be used to solicit entry of vacuum levels, flow rates and/or other information from a user via a touchscreen, a keypad, buttons on the medical waste fluid collection cart 102, and/or another electromechanical input device (e.g., a dial interface).

The communication interface 240 may include one or more communication interfaces for allowing the controller 210 to communicate with one or more other devices, such as via a communication link 247. For example, the communication interface 240 may include a communication interface that allows the medical waste fluid collection cart 102 to communicate with the evacuation station 104 via the coupler 146. In some cases, one or more connectors associated with the communication link 247 may be included within the coupler 146. In other cases, the communication link may be separate from the coupler 146. In some cases, the communication interface 240 may include one or more wired and/or wireless communication interfaces, such as an Ethernet port, a wireless port, an RS-232 port, an RS-422 port, an RS-485 port, and the like. In such cases, the communication interface 240 may allow data entry, reprogramming, upgrading, debugging, and/or other operations to be done remotely, such as by an authorized user, via the communication link 247.

FIG. 3 shows an illustrative user interface screen 300 for providing a user interlock for the medical waste fluid collection cart of FIG. 1. The illustrative user interface screen 300 may be configured to present information to a user, such as for providing a user interlock screen upon power up of a medical waste fluid collection cart. The user interface screen 300 may present information to a user in one or more distinct sections, such as a title section 305, an informational section 320 and/or a data solicitation section 330. For example, the illustrative user interface screen 300 may be configured to solicit information (e.g., a vacuum level, a flow rate, etc.) from a user as part of a user interlock procedure upon power-up of the medical waste fluid collection cart 102. For example, the title section 305 and the informational section 320 may be used to describe why a user is viewing the page and/or other information regarding the reason the screen was presented to the user. Warnings, information about the previous operation of the medical waste fluid collection cart 102 (e.g., a programmed/preset flow and/or vacuum rate, the time of operation, a programmed/preset vacuum level, volume of fluid collected, etc.) and/or information about a failure to complete a previous operation (e.g., power outage information, information about an improper shutdown procedure, a failure to reset a volume and/or vacuum level, etc.) may be shown. In the data solicitation section 330, one or more selection fields or buttons 340, 350 may be presented to a user, for example. For example, the user interface screen 300 may be used for providing a user interface to facilitate user entry of one or more vacuum levels for use by the medical waste fluid collection and disposal system 100. The user interface screen 300 may be configured to receive a user selected vacuum level via the user interface 235, followed by storing the user entered vacuum level in a memory 220, 232 for use by the medical waste fluid collection and disposal system 100.

In some cases, it may be desired to use a default or otherwise predetermined vacuum level and/or flow rate upon start up of the medical waste fluid collection cart 102. For example, a user may desire the medical waste fluid collection cart 102 to use a low programmed/preset vacuum level (e.g., at or near zero mm Hg). In such cases, the user interface screen 300 may be configured to solicit user confirmation that warnings and/or other information, such as the programmed/preset vacuum level, was presented to the user such as in the informational section 320 or in the data solicitation section 330. In some cases, the user confirmation screen 300 may be used for programming or otherwise selecting a default, preset or user defined vacuum level and/or flow rate, such as by selecting a value from one or more presented values or by using a user interface device (e.g., a keyboard, buttons, a dial, a slider, etc.) to select a desired value. In some instances, the medical waste fluid collection cart 102 may automatically revert or go to a programmed/preset low vacuum level (e.g., at or near zero mm Hg) upon start up of the medical waste fluid collection cart 102 without user input or confirmation (e.g., without the user selecting a desired vacuum level). The user may then manually adjust (e.g., increase or decrease) the vacuum level from the programmed/preset low vacuum level to a desired vacuum level, as desired.

The selection fields or buttons 340, 350 may be associated with possible selections available to the user. For example, the first selection field or button 340 may correspond to a first vacuum level, such as a vacuum level associated with a passive collection operation. As shown, the selection field or button 340 may include descriptive text and/or an indication 362 of an associated vacuum level (e.g., a vacuum level within the range of about 0 mm Hg to about 70 mm Hg). The second selection field or button 350 may correspond to a second vacuum level, such as a vacuum level associated an immediately preceding use of the medical fluid collection cart 102. Similarly, the selection field or button 350 may include descriptive text and/or an indication 360 corresponding to a vacuum level associated with the previously programmed vacuum level (e.g., the last known vacuum level used prior to an error condition). In some cases, only one selection field 340 may be shown, such as when it has been decided to use a predetermined vacuum level after power has been applied to the medical waste fluid collection cart 102. In such cases, the selection field 340 may display the value of the predetermined vacuum level. In some cases, the selection field 340 may solicit user input by displaying a prompt (e.g., 'OK'). In some cases, the user interface screen may not include either selection field 340 or selection field 350. Rather, information about the currently active vacuum level setting may be displayed to the user using the title section 305 and/or the informational section 320. In such cases, the user may be prompted to confirm this setting using a user entry device (e.g., a keyboard, a rotary dial, a switch, a button, etc.) before continuing allowing the vacuum level to become active. In some cases, the user interface screen 300 may not be displayed or may not be active for a predetermined duration (e.g., 10 seconds, 15 seconds, etc.).

In some cases, a user interlock may be performed using one or more user entry devices. For example, the medical waste fluid collection cart 102 may be configured to be set at a predetermined/preset vacuum level or flow rate (e.g., at or near zero mm Hg) upon start-up until a user enters a new vacuum level. In some cases, the newly entered vacuum level may become active immediately after entry by a user. However, in many cases, the user may be prompted to confirm the newly entered vacuum level setting before use. For example, a display may prompt the user to confirm the new vacuum level setting, such as by pressing a button (e.g., a mechanical button, a button on a touch screen, etc.), flipping a switch, depressing a key, turning a dial, and the like.

In some cases, the predetermined/preset vacuum level may remain active until a user performs a predetermined sequence of events. For example, the controller of the medical waste fluid collection cart may be programmed to use the predetermined/preset vacuum level after a successful power up sequence. In some cases, the controller may be programmed to override a programmed vacuum level setting until the user performs the predetermined sequence of events. For example, the user may be prompted or otherwise required to depress keys or buttons in a particular sequence. In some cases, the user may be required to move a mechanical entry device (e.g., a dial, a slider, a multi-position switch, etc.) to a particular position before selecting a vacuum level different than the preprogrammed/preset vacuum level. For example, a user may be prompted, or otherwise may be required, to turn a dial from the previously programmed setting (e.g., vacuum level, flow rate, etc.) to a particular position or to a particular vacuum level/flow rate setting (e.g., about zero mm Hg) before turning the dial to the desired vacuum level. Of course, these examples are merely illustrative and are not meant to preclude other similar methods or procedures from being used.

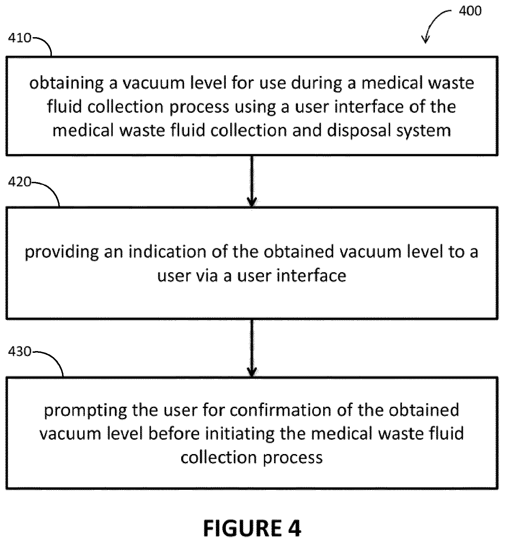

FIG. 4 shows an illustrative technique of providing a user interlock for configuring a vacuum level setting in the medical waste fluid collection cart of FIG. 1. For example, at 410 the controller 210 may be configured for initiating a startup procedure of the medical waste fluid collection and disposal system upon turning on the controller 210. At 420, the controller 210 may be configured to determine whether an improper shutdown of the medical waste fluid collection and disposal system 100 immediately preceded the initiated startup procedure. If, at 430, the controller identifies an improper shutdown of the medical waste fluid collection and disposal system 100, the controller 210 may cause a user interface screen 300 to be displayed to a user during the startup procedure. For example, the user interface screen 300 may be configured for prompting the user to select a vacuum level for use by the medical fluid waste collection and disposal system 100 by selecting either a predetermined/preset vacuum level or a previously programmed vacuum level associated with a previous fluid collection process immediately preceding the last loss of power to the controller 210. Until the user makes a selection of one of the options from the interface screen 300 (e.g., the predetermined/preset vacuum level or the previously programmed vacuum level associated with a previous fluid collection process immediately preceding the last loss of power to the controller), the vacuum pump may remain off and/or zero or a low amount of suction (e.g., less than 5 mm Hg, less than 10mm Hg) may be present in the waste canister or cylinder 112 and/or suction tubing to the patient. Once the user makes a selection of the desired vacuum level from the interface screen 300, the controller 210 may turn on the vacuum pump and/or send a signal to the vacuum pump and/or valve, for example, to increase the vacuum level in the waste canister or cylinder 112 and/or suction tubing to the patient to the desired level. Thus, the startup procedure may prevent a vacuum level above a threshold level in the waste canister or cylinder 112 and/or suction tubing to the patient until after the user-inputted response to the startup confirmation screen has been provided.

Having thus described several illustrative embodiments of the present disclosure, those of skill in the art will readily appreciate that yet other embodiments may be made and used within the scope of the claims hereto attached.

## Claims

1. A medical waste fluid collection cart including a graphical user interface for allowing a user to operate or monitor one or more functions of the medical waste fluid collection cart, the medical waste fluid collection cart comprising:
at least one fluid collection cylinder;
a manifold configured to allow for fluid transfer into and/or out of the at least one fluid collection cylinder;
a suction tube in fluid communication with the at least one fluid collection cylinder, the suction tube for providing a fluid path to the at least one fluid collection cylinder for medical waste fluid;
a vacuum port in fluid communication with the at least one fluid collection cylinder, the vacuum port configured to connect the at least one fluid collection cylinder to a vacuum source to provide a vacuum level in the fluid collection cylinder for use in collecting a medical waste fluid, wherein the vacuum level in the at least one fluid collection cylinder is adjustable;
a controller for controlling one or more functions of the medical waste fluid collection cart including providing a user interface device to select the vacuum level provided in the suction tube;
a memory coupled to the controller, the memory configured for storing one or more vacuum levels; and
a graphical user interface communicatively coupled to the controller, the graphical user interface configured to allow a user to monitor and/or control one or more functions of the medical waste fluid collection cart, **characterised in that** the graphical user interface includes:
a user interlace screen configured to display one of more visual warnings to the user, during-start up, -regarding- vacuum-or the use of high flow suction for procedures that do not require it, and to solicit confirmation from the user that the warning was presented.

2. The medical waste fluid collection cart of claim 1, wherein the user interface screen is displayed to a user during a startup procedure following an improper shutdown of the medical waste fluid collection cart

3. The medical waste fluid collection cart of claim 1, wherein the graphical user interface includes a user confirmation screen prompting the user to confirm the selected vacuum level.

4. The medical waste fluid collection cart of claim 1, wherein the controller is configured to delay activating a vacuum in the suction tube until the user confirms the selected vacuum level.

5. The medical waste fluid collection cart of claim 1, wherein the screen includes a first selection corresponding to a predetermined low vacuum level, and a second selection corresponding to a previously used vacuum level.

6. The medical waste fluid collection cart of claim 1, wherein the controller enables a predetermined low vacuum level or a previously used vacuum level upon power up of the medical waste fluid collection cart.

7. The medical waste fluid collection cart of claim 1, wherein upon initiating a startup procedure, the controller is configured to set a predetermined vacuum level until the user enters a new vacuum level setting.

8. The medical waste fluid collection cart of claim 7, wherein the controller is configured to override the new vacuum level setting until the user performs a predetermined sequence of events.

9. The medical waste fluid collection cart of claim 1, wherein the memory i s configured for instructions to monitor a shutdown procedure of the medical waste fluid collection cart, wherein the controller is configured to process the instructions to determine an indication of an improper shutdown of the medical waste fluid collection cart.

10. The medical waste fluid collection cart of claim 9, wherein the indication of an improper shutdown of the medical waste fluid collection cart includes an indication of a power loss condition and/or an indication of an improper shutdown procedure.

11. The medical waste fluid collection cart of claim 10, wherein the controller is configured to:
store a vacuum level associated with an active medical waste fluid collection process in the memory;
store an indication whether the active medical waste fluid collection process completes successfully in the memory; and
wherein the indication of a successful completion of the active medical waste fluid collection process is used to display the user confirmation screen before beginning a subsequent medical waste fluid collection process.

12. A method for soliciting user confirmation of a selected vacuum level for a medical waste fluid collection and disposal system, the method comprising:
soliciting information from a user, the information including a vacuum level for use during a medical waste fluid collection process, by use of a user interface screen (300) of the medical waste fluid collection and disposal system;
displaying an indication of the solicitedvacuum level to the user via the user interface screen (300) soliciting and obtaining confirmation from the user of the vacuum level before initiating the medical waste fluid collection process; **characterised by**
displaying one or more warnings via the user interfacescreen (300) to the user, during start up, regarding vacuum or the use of high flow suction for procedures that do not require it; and via the user interface screen (300) to the use soliciting confirmation from the user that the one or more warnings were presented

13. The method of claim 12, further comprising: delaying activation of a vacuum in a suction tube of the medical waste fluid collection and disposal system until the user confirms the selected vacuum level.

14. The method of claim 12, further comprising: displaying a notification to the user via the user interface during a startup procedure to select either a predetermined vacuum level or a previously programmed vacuum level associated with a previous fluid collection process.

15. The method of claim 14, wherein the notification is displayed during a startup procedure directly following an improper shutdown of the medical waste fluid collection and disposal system.

## Patentansprüche

1. Sammelwagen für medizinische Fluidabfälle, der eine grafische Benutzeroberfläche umfasst, um es einem Benutzer zu ermöglichen, eine oder mehrere Funktionen des Sammelwagens für medizinische Fluidabfälle zu bedienen oder zu überwachen, wobei der Sammelwagen für medizinische Fluidabfälle folgendes umfasst:
mindestens einen Fluidsammelzylinder;
einen Verteiler, der so gestaltet ist, dass er eine Fluidübertragung in den und/oder aus dem mindestens einen Fluidsammelzylinder ermöglicht;
ein Saugrohr in Fluidkommunikation mit dem mindestens einen Fluidsammelzylinder, wobei das Saugrohr einen Fluidweg zu dem mindestens einen Fluidsammelzylinder für medizinische Fluidabfälle bereitstellt;
einen Vakuumanschluss in Fluidkommunikation mit dem mindestens einen Fluidsammelzylinder, wobei der Vakuumanschluss so gestaltet ist, dass er den mindestens einen Fluidsammelzylinder mit einer Vakuumquelle verbindet, um eine Vakuumhöhe in dem Fluidsammelbehälter zur Verwendung beim Sammeln eines medizinischen Fluidabfalls bereitzustellen, wobei die Vakuumhöhe in dem mindestens einen Fluidsammelzylinder einstellbar ist;
eine Steuereinheit zum Steuern einer oder mehrerer Funktionen des Sammelwagens für medizinische Fluidabfälle, umfassend das Bereitstellen einer Benutzeroberfläche zur Auswahl der in dem Saugrohr bereitgestellten Vakuumhöhe;
einen Speicher, der mit der Steuereinheit gekoppelt ist, wobei der Speicher zum Speichern einer oder mehrerer Vakuumhöhen gestaltet ist; und
eine grafische Benutzeroberfläche, die kommunikationsfähig mit der Steuereinheit gekoppelt ist, wobei die grafische Benutzeroberfläche so gestaltet ist, dass sie es einem Benutzer ermöglicht, eine oder mehrere Funktionen des Sammelwagens für medizinische Fluidabfälle zu überwachen und/oder zu steuern, **dadurch gekennzeichnet, dass** die grafische Benutzeroberfläche folgendes umfasst:
einen Benutzeroberflächenbildschirm, der so gestaltet ist, dass er dem Benutzer während der Inbetriebnahme eine oder mehrere visuelle Warnhinweise in Bezug auf das Vakuum oder den Einsatz eines Saugbetriebs mit hoher Strömungsrate für Prozeduren anzeigt, die dies nicht erfordern, sowie eine Bestätigung von dem Benutzer anfordert, dass der Warnhinweis angezeigt worden ist.

2. Sammelwagen für medizinische Fluidabfälle nach Anspruch 1, wobei der Benutzeroberflächenbildschirm einem Benutzer während einer Inbetriebnahmeprozedur angezeigt wird, die auf eine unvorschriftsmäßige Abschaltung des Sammelwagens für medizinische Fluidabfälle folgt.

3. Sammelwagen für medizinische Fluidabfälle nach Anspruch 1, wobei die grafische Benutzeroberfläche einen Benutzerbestätigungsbildschirm umfasst, der den Benutzer auffordert, die gewählte Vakuumhöhe zu bestätigen.

4. Sammelwagen für medizinische Fluidabfälle nach Anspruch 1, wobei die Steuereinheit so gestaltet ist, dass sie die Aktivierung eines Vakuums in dem Saugrohr verzögert, bis der Benutzer die ausgewählte Vakuumhöhe bestätigt.

5. Sammelwagen für medizinische Fluidabfälle nach Anspruch 1, wobei der Bildschirm eine erste Auswahl umfasst, die einer vorbestimmten niedrigen Vakuumhöhe entspricht, und eine zweite Auswahl, die einer zuvor verwendeten Vakuumhöhe entspricht.

6. Sammelwagen für medizinische Fluidabfälle nach Anspruch 1, wobei die Steuereinheit beim Hochfahren des Sammelwagens für medizinische Fluidabfälle eine vorbestimmte niedrige Vakuumhöhe oder eine zuvor verwendete Vakuumhöhe aktiviert.

7. Sammelwagen für medizinische Fluidabfälle nach Anspruch 1, wobei die Steuereinheit so gestaltet ist, dass sie nach dem Einleiten einer Inbetriebnahmeprozedur eine vorbestimmte Vakuumhöhe einstellt, bis der Benutzer eine neue Einstellung für die Vakuumhöhe eingibt.

8. Sammelwagen für medizinische Fluidabfälle nach Anspruch 7, wobei die Steuereinheit so gestaltet ist, dass sie die neue Vakuumhöhe übersteuert, bis der Benutzer eine vorbestimmte Sequenz von Ereignissen ausführt.

9. Sammelwagen für medizinische Fluidabfälle nach Anspruch 1, wobei der Speicher gestaltet ist für Anweisungen zur Überwachung einer Abschaltprozedur des Sammelwagens für medizinische Fluidabfälle, wobei die Steuereinheit so gestaltet ist, dass sie die Anweisungen verarbeitet, um einen Hinweis auf eine unvorschriftsmäßige Abschaltung des Sammelwagens für medizinische Fluidabfälle zu bestimmen.

10. Sammelwagen für medizinische Fluidabfälle nach Anspruch 9, wobei der Hinweis auf eine unvorschriftsmäßige Abschaltung des Sammelwagens für medizinische Fluidabfälle einen Hinweis auf einen Energieverlustzustand und/oder einen Hinweis auf eine unvorschriftsmäßige Abschaltprozedur umfasst.

11. Sammelwagen für medizinische Fluidabfälle nach Anspruch 10, wobei die Steuereinheit für folgende Zwecke gestaltet ist:
Speichern einer Vakuumhöhe, die einem aktiven Sammelprozess für medizinische Fluidabfälle zugeordnet ist, in dem Speicher;
Speichern eines Hinweises, ob der aktive Sammelprozess für medizinische Fluidabfälle erfolgreich abgeschlossen worden ist, in dem Speicher; und
wobei der Hinweis auf einen erfolgreichen Abschluss des aktiven Sammelprozesses für medizinische Fluidabfälle verwendet wird, um den Benutzerbestätigungsbildschirm vor dem Beginn eines folgenden Sammelprozesses für medizinische Fluidabfälle anzuzeigen.

12. Verfahren zur Anforderung einer Benutzerbestätigung einer ausgewählten Vakuumhöhe für ein System zum Sammeln und Entsorgen medizinischer Fluidabfälle, wobei das Verfahren folgendes umfasst:
Anfordern von Informationen von einem Benutzer, wobei die Informationen eine Vakuumhöhe zur Verwendung während einem Sammelprozesses für medizinische Fluidabfälle umfassen, unter Verwendung eines Benutzerschnittstellenbildschirms (300) des Systems zum Sammeln und Entsorgen medizinischer Fluidabfälle;
Anzeigen eines Hinweises auf die angeforderte Vakuumhöhe an den Benutzer über den Benutzerschnittstellenbildschirm (300), wobei die Bestätigung der Vakuumhöhe von dem Benutzer vor Einleitung des Sammelprozesses für medizinische Fluidabfälle angefordert und erhalten wird; **gekennzeichnet durch**
Anzeigen eines oder mehrerer Warnhinweise über den Benutzerschnittstellenbildschirm (300) an den Benutzer während der Inbetriebnahme in Bezug auf das Vakuum oder den Einsatz eines Saugbetriebs mit hoher Strömungsrate für Prozeduren, die dies nicht erfordern; sowie über den Benutzerschnittstellenbildschirm (300), Anfordern einer Bestätigung von dem Benutzer, dass der eine oder die mehreren Warnhinweise angezeigt worden sind.

13. Verfahren nach Anspruch 12, ferner umfassend:
Verzögern der Aktivierung eines Vakuums in einem Saugrohr des Systems zum Sammeln und Entsorgen medizinischer Fluidabfälle, bis der Benutzer die ausgewählte Vakuumhöhe bestätigt.

14. Verfahren nach Anspruch 12, ferner umfassend:
Anzeigen einer Mitteilung an den Benutzer über die Benutzeroberfläche während einer Inbetriebnahmeprozedur zur Auswahl entweder einer vorbestimmten Vakuumhöhe oder einer zuvor programmierten Vakuumhöhe, die einem vorherigen Fluidsammelprozess zugeordnet ist.

15. Verfahren nach Anspruch 14, wobei die Mitteilung während einer Inbetriebnahmeprozedur unmittelbar nach einer unvorschriftsmäßigen Abschaltung des Systems zum Sammeln und Entsorgen medizinischer Fluidabfälle angezeigt wird.

## Revendications

1. Chariot de collecte de fluides de déchets médicaux comprenant une interface utilisateur graphique pour permettre à un utilisateur de faire fonctionner ou de surveiller au moins une fonction du chariot de collecte de fluides de déchets médicaux, le chariot de collecte de fluides de déchets médicaux comprenant :
au moins un cylindre de collecte de fluides ;
un collecteur conçu pour permettre le transfert de fluide dans et/ou hors de l'au moins un cylindre de collecte de fluides ;
un tube d'aspiration en communication fluidique avec au moins un cylindre de collecte de fluides, le tube d'aspiration servant à fournir un chemin de fluide vers l'au moins un cylindre de collecte de fluides pour les fluides de déchets médicaux ;
un orifice à vide en communication fluidique avec l'au moins un cylindre de collecte de fluides, l'orifice à vide étant conçu pour relier l'au moins un cylindre de collecte de fluides à une source de vide afin de fournir un niveau de vide dans le cylindre de collecte de fluides pour une utilisation dans la collecte d'un fluide de déchets médicaux, le niveau de vide dans l'au moins un cylindre de collecte de fluides étant réglable ;
un dispositif de commande permettant de commander au moins une fonction du chariot de collecte de fluides de déchets médicaux, y compris de fournir un dispositif d'interface utilisateur pour sélectionner le niveau de vide fourni dans le tube d'aspiration ;
une mémoire couplée au dispositif de commande, la mémoire étant conçue pour stocker au moins un niveau de vide ; et
une interface utilisateur graphique couplée en communication au dispositif de commande, l'interface utilisateur graphique étant conçue pour permettre à un utilisateur de surveiller et/ou de commander au moins une fonction du chariot de collecte de fluides de déchets médicaux, **caractérisé en ce que** l'interface utilisateur graphique comprend :
un écran d'interface utilisateur conçu pour afficher au moins un avertissement visuel à l'utilisateur, lors du démarrage, concernant le vide ou l'utilisation de l'aspiration à haut débit pour des procédures qui ne le nécessitent pas, et pour demander à l'utilisateur de confirmer que l'avertissement a été présenté.

2. Chariot de collecte de fluides de déchets médicaux selon la revendication 1, l'écran d'interface utilisateur étant affiché à un utilisateur pendant une procédure de démarrage à la suite d'un arrêt incorrect du chariot de collecte de fluides de déchets médicaux.

3. Chariot de collecte de fluides de déchets médicaux selon la revendication 1, l'interface utilisateur graphique comprenant un écran de confirmation invitant l'utilisateur à confirmer le niveau de vide sélectionné.

4. Chariot de collecte de fluides de déchets médicaux selon la revendication 1, le dispositif de commande étant conçu pour retarder l'activation d'un vide dans le tube d'aspiration jusqu'à ce que l'utilisateur confirme le niveau de vide sélectionné.

5. Chariot de collecte de fluides de déchets médicaux selon la revendication 1, l'écran comprenant une première sélection correspondant à un niveau de vide bas prédéfini, et une seconde sélection correspondant à un niveau de vide utilisé précédemment.

6. Chariot de collecte de fluides de déchets médicaux selon la revendication 1, le dispositif de commande permettant un niveau de vide bas prédéfini ou un niveau de vide précédemment utilisé lors de la mise sous tension du chariot de collecte de fluides de déchets médicaux.

7. Chariot de collecte de fluides de déchets médicaux selon la revendication 1, lors du lancement d'une procédure de démarrage, le dispositif de commande étant conçu pour régler un niveau de vide prédéfini jusqu'à ce que l'utilisateur entre un nouveau réglage de niveau de vide.

8. Chariot de collecte de fluides de déchets médicaux selon la revendication 7, le dispositif de commande étant conçu pour passer outre le nouveau réglage du niveau de vide jusqu'à ce que l'utilisateur effectue une séquence d'événements prédéfinie.

9. Chariot de collecte de fluides de déchets médicaux selon la revendication 1, la mémoire étant conçue pour des instructions pour surveiller une procédure d'arrêt du chariot de collecte de fluides de déchets médicaux, le dispositif de commande étant conçu pour traiter les instructions afin de déterminer une indication d'un arrêt incorrect du chariot de collecte de fluides de déchets médicaux.

10. Chariot de collecte de fluides de déchets médicaux selon la revendication 9, l'indication d'un arrêt incorrect du chariot de collecte de fluides de déchets médicaux comprenant une indication d'une condition de perte de puissance et/ou une indication d'une procédure d'arrêt incorrect.

11. Chariot de collecte de fluides de déchets médicaux selon la revendication 10, le dispositif de commande étant conçu pour :
stocker dans la mémoire un niveau de vide associé à un processus de collecte de fluides de déchets médicaux actif ;
stocker dans la mémoire une indication indiquant si le processus de collecte de fluides de déchets médicaux actif s'achève avec succès ; et
l'indication d'un achèvement réussi du processus de collecte de fluide de déchets médicaux actif étant utilisée pour afficher l'écran de confirmation de l'utilisateur avant de commencer un processus de collecte de fluides de déchets médicaux subséquents.

12. Procédé pour solliciter la confirmation par l'utilisateur d'un niveau de vide sélectionné pour un système de collecte et d'élimination de fluides de déchets médicaux, le procédé comprenant les étapes consistant à :
solliciter des informations auprès d'un utilisateur, les informations comprenant un niveau de vide à utiliser lors d'un processus de collecte de fluides de déchets médicaux, à l'aide d'un écran d'interface utilisateur (300) du système de collecte et d'élimination de fluides de déchets médicaux ;
afficher une indication du niveau de vide sollicité à l'utilisateur par l'intermédiaire de l'écran d'interface utilisateur (300), solliciter et obtenir une confirmation par l'utilisateur du niveau de vide avant de lancer le processus de collecte de fluides de déchets médicaux ; **caractérisé par** les étapes consistant à
afficher au moins un avertissement par l'intermédiaire de l'écran d'interface utilisateur (300) à l'utilisateur, lors du démarrage, concernant le vide ou l'utilisation de l'aspiration à haut débit pour des procédures qui ne le nécessitent pas ; et par l'intermédiaire de l'écran d'interface utilisateur (300),
demander à l'utilisateur de confirmer que l'au moins un avertissement a été présenté à l'utilisateur.

13. Procédé selon la revendication 12, comprenant en outre l'étape consistant à :
retarder l'activation d'un vide dans un tube d'aspiration du système de collecte et d'élimination de fluides de déchets médicaux jusqu'à ce que l'utilisateur confirme le niveau de vide choisi.

14. Procédé selon la revendication 12, comprenant en outre l'étape consistant à :
afficher une notification à l'utilisateur par l'intermédiaire de l'interface utilisateur pendant une procédure de démarrage pour sélectionner soit un niveau de vide prédéfini, soit un niveau de vide précédemment programmé associé à un processus de collecte de fluides antérieur.

15. Procédé selon la revendication 14, la notification étant affichée pendant une procédure de démarrage suivant directement un arrêt incorrect du système de collecte et d'élimination de fluides de déchets médicaux.
